# EUROPEAN PATENT APPLICATION

(11) **EP 4 755 418 A1**
(43) Date of publication of application: **10.06.2026**
(21) Application number: 24307041.4
(22) Date of filing: 05.12.2024
(51) Int. Cl.: A61M 5/315, A61M 5/31

(54) **DOSING SYSTEM, ASSOCIATED ASSEMBLY AND ASSOCIATED INJECTION DEVICE**

(71) Applicant: NEMERA LA VERPILLIERE, 38290 La Verpillière (FR); Nemera Insight Chicago, Chicago, IL 60640 (US)
(72) Inventor: BELTON, Antonio J., RICHTON PARK, 60471 (US); Cerqueira, Guillaume, 69008 LYON (FR); HUET, Gildas, 69230 SAINT-GENIS-LAVAL (FR)
(74) Representative: Lavoix

(57) **Abstract**

A dosing system (75) for an injection device (1;1001), the injection device comprising a syringe barrel (10) and a plunger rod (50), the dosing system comprising a main part (80) configured to be assembled on the syringe barrel, the main part comprising a body defining a receiving volume (90), and a sliding part (165) mounted on the main part and configured to slide relative to the main part between an initial position and an activated position. When the main part is assembled on the syringe barrel, the receiving volume (90) is configured to receive at least partially the plunger rod, and the sliding part is configured to cooperate with the plunger rod when sliding from the initial position to the activated position, so that the sliding part pushes the plunger rod in translation relative to the body, from a first position to a second position, in an injection direction (D).

## Description

The present invention relates to a dosing system, notably for delivering a precise dose of a fluid for medical or pharmaceutical applications. The present invention further relates to an assembly of such a dosing system with a plunger rod, and to an injection device comprising such an assembly.

Injection devices, such as syringes, are known to inject determined doses of fluid to a patient, notably through parenteral way. A syringe typically comprises a syringe barrel on which an injection needle is attached. The syringe barrel defines a container for receiving a fluid to inject. A plunger cooperates with the syringe barrel to push the fluid from the container towards the injection needle and then inject the fluid to a patient through the needle. Usually, the plunger is moved compared to the syringe barrel by a pressure of the user on a plunger head.

Before the fluid injection step, a purging step can be needed to eject excess fluid or air bubbles trapped into the container or inside the needle. This purging step participates to a precise dosing of the injected fluid and reduces the risk to inject air bubbles to a patient.

For some applications and in particular medical applications, the accuracy of the dose of fluid to inject is particularly important. It is the case for ophthalmic injections, intraocular injections, and notably intravitreal injections. Indeed, the eye being a small and closed environment, such injections could lead to unwanted intraocular pressure increase if not well controlled. Moreover, the injection volumes can be as low as 0.03 mL to 0.1 mL, which also requires a precise dosing.

The use of pre-filled syringes, also called PFS, is preferred for some applications like intraocular injections, notably because it decreases the risk of microbial and particle contamination of the fluid to inject compared to syringes that require the user to perform a filling step in which the fluid is sucked from an external container via the needle. Moreover, the use of PFS reduces the preparation time of the injection.

The existing pre-filled syringes are generally designed for volumes higher or equal to 0.5ml and according to standards, like the ISO 11040-4 for syringe barrels.

Generally, a syringe, and notably a PFS, is equipped with a graduation on the syringe barrel for the user to visually align with a part of the plunger, generally a part of a plunger stopper disposed at the end of the plunger, and thus select the dose of fluid to inject. However, this kind of dosing system depends on the user interpretation of this alignment, which may lead to a lack of accuracy. This risk is particularly important when a syringe designed for 0.5ml to 1ml injection volumes is used for a lower injection volume.

It is then sought to provide an accurate dosing system, notably able to cooperate with pre-filled syringes, and which delivers a precise dose in a user-independent way.

Another objective of the invention is to provide an accurate dosing system for injection volumes lower than 0.4ml and able to cooperate with a syringe barrel initially designed for injection volumes higher or equal to 0.5ml.

To this end, aspects of the invention pertain to a dosing system for an injection device, the injection device comprising a syringe barrel and a plunger rod extending along a rod axis, the syringe barrel extending along a first axis, the dosing system comprising:
- a main part configured to be assembled on the syringe barrel of the injection device, the main part comprising a body defining a receiving volume which extends around a second axis and
- a sliding part mounted on the main part and configured to slide relative to the main part along a third axis, which intersects the second axis, between an initial position and an activated position,
wherein, when the main part is assembled on the syringe barrel of the injection device, the receiving volume is configured to receive at least partially the plunger rod so that the second axis, the rod axis and the first axis are superimposed, and the sliding part is configured to cooperate with the plunger rod when sliding along the third axis from the initial position to the activated position, so that the sliding part pushes the plunger rod in translation relative to the body, from a first position to a second position, in an injection direction, extending along the rod axis.

Thanks to the invention, in particular thanks to the sliding part, the displacement of the plunger rod is defined by the dosing system thus being user-independent. Indeed, the dosing system constrains the translation of the plunger rod from the first position to the second position. The second position of the plunger rod, which corresponds for example to a position of end of purging step and/or start of injection step, is then only determined by the dosing system and does not depend on any user interpretation.

According to advantageous but optional aspects, such a dosing system may incorporate one or more of the following features, considered alone or according to any technically allowable combination:
Optionally in some examples, including in at least one preferred example, the main part comprises at least one first guiding element and the sliding part comprises at least one second guiding element, the at least one first guiding element cooperating with the at least one second guiding element to guide the sliding of the sliding part along the third axis between the initial position and the activated position. Preferably, a first element of the at least one first guiding element and the at least one second guiding element comprises a groove extending parallel to the third axis and a second element of the at least one first guiding element and the at least one second guiding element comprises a protrusion, the protrusion being configured to slide within the groove to guide the sliding of the sliding part along the third axis between the initial position and the activated position

A technical benefit may include guiding the trajectory of the sliding part so that the user could slide the sliding part easily while reducing the risk of wrong trajectory.

Optionally in some examples, including in at least one preferred example, the groove comprises at least one notch configured to receive the protrusion so as to stabilize the sliding part in the initial position and/or in the activated position. Preferably, the groove is located on an elastic tongue, the tongue being configured to elastically deform to allow the protrusion to enter and exit the at least one notch.

A technical benefit may include stabilizing the sliding part in the in initial and/or activated position. Then, the user must apply a certain force to slide the sliding part from the initial position and/or from the activated position which avoids unintended sliding from the initial position towards the activated position and/or from the activated position towards the initial position. Moreover, the user gets feedback when the sliding part reaches the activated position.

Optionally in some examples, including in at least one preferred example, the sliding part comprises a first stop configured to block the plunger rod in the first position when the sliding part is in the initial position. A technical benefit may include preventing the displacement of the plunger when the sliding part in the initial position. In other terms, there is no risk that the user moves unwittingly the plunger rod by pressing the plunger flange and spoils a part or the totality of the fluid.

Optionally in some examples, including in at least one preferred example, the sliding part comprises a second stop configured, when the sliding part is in the activated position, to prevent the plunger rod from being translated along the injection direction beyond a third position of the plunger rod, the third position being beyond the second position along the injection direction. A technical benefit may include improving the accuracy of the injection dose. Indeed, the stoke between the second and the third positions of the plunger rod defines an accurate volume of fluid injected which depends only on the dimensions of the different elements of the injection device. Thus, the volume of fluid injected is accurate and does not depend on the user's appreciation.

Optionally in some examples, including in at least one preferred example, the main part comprises a housing configured to receive a barrel flange of the syringe barrel, the housing comprising:
- a first arm configured to be in contact with a distal surface of the barrel flange,
- a second arm secured to the body of the main part and configured to lean against a proximal surface of the barrel flange, opposite to the distal surface, the second arm being linked to the first arm and mobile along a direction parallel to the second axis with respect to the first arm, and
- an elastic element, configured to exert a force onto the first arm and the second arm tending to bring the second arm towards the first arm along a direction parallel to the second axis.

Preferably, the elastic element links the second arm to the the first arm. Preferably, the elastic element is a spring, preferably a helicoidally spring or a leaf spring, or an elastic plastic layer. Preferably, the first arm, the second arm and the elastic element are formed of a single part. A technical benefit may include adapting the dosing system to the barrel flange. Indeed, the barrel flange is blocked in the housing so that the syringe barrel cannot move relative to the dosing system along the first axis. Thus, the risk of dosage inaccuracy due to displacement of elements relative to one another is reduced. This is particularly advantageous with syringe barrel made of glass, for example according to ISO 11040-4 standard, which could have big tolerances in the barrel flange thickness.

Optionally in some examples, including in at least one preferred example, the second axis is orthogonal to the third axis. Optionally in some examples, including in at least one preferred example, the main part comprises a first contact surface and the sliding part comprises a second contact surface, opposed to the first contact surface along the third axis, the first and second contact surfaces being adapted to receive a finger of a user and being configured to allow the user to slide the sliding part. A technical benefit may include facilitating the displacement of the sliding part relative to the main part.

Optionally in some examples, including in at least one preferred example, the sliding part comprises a ramp, inclined by a first non-zero angle with respect to the third axis and inclined by a second non-zero angle with respect to the second axis, the ramp being configured to cooperate with the plunger rod so as to push the plunger rod in translation relative to the syringe barrel in the injection direction, from the first position to the second position, when the main part is assembled on the syringe barrel of the injection device and when the sliding part is sliding along the third axis from the initial position to the activated position. A technical benefit may include easily transforming the displacement of a sliding part in a displacement of the plunger in the injection direction.

The invention also relates to an assembly a dosing system, as described above, and a plunger rod, the plunger rod extending along a rod axis, the plunger rod being configured to be received at least partially in the receiving volume and to translate relative to the body from a first position to a second position in the injection direction extending along the rod axis. Preferably, the sliding part comprises a first pushing element, and the plunger rod comprises a rod portion, extending mainly along the rod axis, and a second pushing element configured to cooperate with the first pushing element so that, when the sliding part is sliding along the third axis from the initial position to the activated position, the plunger rod is pushed in the injection direction by the first pushing element from the first position to the second position. Preferably, the first pushing element is a ramp, inclined by a first non-zero angle with respect to the third axis and inclined by a second non-zero angle with respect to the second axis, and the second pushing element is a pin, protruding from the rod portion along a pin axis distinct from the rod axis. Preferably, the sliding part comprises a second stop, wherein, when the sliding part is in the activated position, the second stop is configured to prevent the plunger rod from being translated along the injection direction beyond a third position of the plunger rod, the third position being beyond the second position along the injection direction, and the second pushing element of the plunger rod is in abutment with the second stop of the dosing system along the injection direction when the plunger rod is in the third position and the sliding part is in the activated position

The invention also relates to an injection device comprising:
- a syringe barrel(10)extending along a first axis and
- an assembly according to any one of the claims 15 to 18,
wherein the main part of the dosing system is assembled on the syringe barrel and the plunger rod is mounted on the syringe barrel and the plunger rod is at least partially received in the receiving volume.

The invention will be better understood, and other advantages thereof will appear more clearly, in light of the following description of an injection device, provided solely as a non-limiting example and done in reference to the appended drawings, in which:
[Fig. 1] Figure 1 is a perspective view of an injection device , the injection device comprising a dosing system according to a first embodiment of the invention , a sliding part of the dosing system being in an initial position and a plunger rod of the injection device being in a first position,
[Fig. 2] Figure 2 is a perspective view of a main part of a dosing system of the injection device of figure 1,
[Fig. 3] Figure 3 is a perspective view of the sliding part of the dosing system of the injection device of figure 1,
[Fig. 4] Figure 4 is another perspective view of the injection device of figure 1,
[Fig. 5] Figure 5 is a cross-section following the plan V of the injection device of figure 4, viewed in perspective,
[Fig. 6] Figure 6 is a cross-section following the plan VI of the injection device of figure 5, the sliding part being in an initial position and the plunger rod being in a first position,
[Fig. 7] Figure 7 is a view similar to figure 1, the sliding part of injection device being in an activated position and the plunger rod being in a third position,
[Fig. 8] Figure 8 is a cross-section following the plan VIII of the injection device of figure 5, viewed in perspective, the sliding part being in an activated position and the plunger rod being in a second position,
[Fig. 9] Figure 9 is a cross-section similar to figure 6 of the injection device of figure 7, the sliding part being in an activated position and the plunger rod being in a second position,
[Fig. 10] Figure 10 is a cross-section similar to figure 9 of the injection device of figure 7, the sliding part being in an activated position and the plunger rod being in a third position,
[Fig. 11] Figure 11 is a functional representation of the injection device of the figure 1 to 10, and
[Fig. 12] Figure 12 is functional representation of an injection device, the injection device comprising a dosing system according to a second embodiment of the invention.

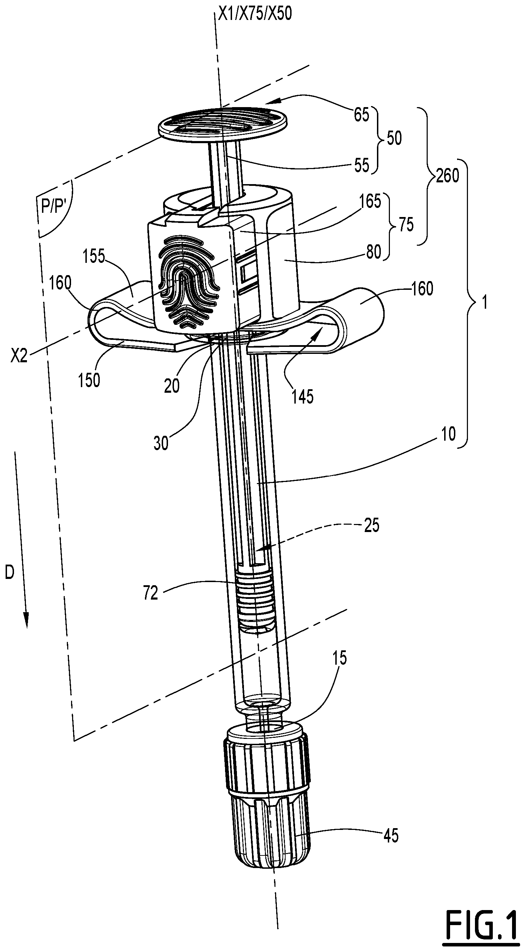

An injection device 1 according to a first embodiment of the invention is represented on figure 1. The injection device 1 is, for example, a medical injection device, in particular an ophthalmic injection device, more particularly an intravitreal injection device. The injection device 1 is configured to inject a fluid to a target, for example to a patient organ, in particular to an eye of a patient. The injection device 1 is, for example, configured to inject a volume of fluid between 0.01 ml to 0.4ml, in particular between 0.03 ml to 0.1ml. The injection device 1 is, for example, configured to inject a single dose of fluid. The injection device 1 is, for example, a medical injection device pre-filled with the fluid. The fluid injected with the injection device 1 comprises for instance, Bevacizumab, Ranibizumab, Brolucizumab, Faricimab, Aflibercept, Pegcetacoplan, Avacincaptad pegol, Triamcinolone acetonide, Ganciclovir, Clindamycin, Foscarnet, Fomivirsen, Methotrexate, Vancomycin, Ceftazidime, Amikacin, Amphotericin, Voriconazole, Pegaptanib, or any formulation of a drug that is suitable for intraocular injection, this list being non-exhaustive.

The injection device 1 comprises a syringe barrel 10. The syringe barrel 10 is a part of revolution around a first axis X1 and extends in an injection direction D from a proximal end 20 to a distal end 15. The injection direction D corresponds to a direction along which the fluid is injected when the injection device 1 is assembled. Hence, the syringe barrel 10 extends along the first axis X1. In this example, the syringe barrel 10 is symmetrical relative to a plan P, which comprises the first axis X1.

The term "distal", when referring to the injection device 1, refers to the injection direction D and refer to element oriented in the direction where the fluid is injected. The term "proximal", when referring to the injection device 1, refers to a direction opposite to the injection direction D. A "distal surface" is oriented in the injection direction D, a "proximal surface" is oriented in the opposite direction of the injection direction D.

The syringe barrel 10is, for example, formed of a single part. The syringe barrel 10 is for example made of glass or a thermoplastic material. The syringe barrel 10 is for example designed according to the ISO 11040-4 standard.

The distal end 15 is configured to be connected to a needle, not shown, for example by the intermediate of a needle connector. The needle is configured to be partially inserted into the target and to carry the fluid to inject from the injection device 1 to the target.

The syringe barrel 10 defines a barrel volume 25. More precisely, an inner surface of the syringe barrel 10 defines the barrel volume 25. The barrel volume 25 runs through the syringe barrel 10 from the distal end 15 to the proximal end 20. The barrel volume 25 is configured to receive the fluid to inject.

The syringe barrel 10 comprises a barrel flange 30 positioned at the proximal end 20. The barrel flange 30 extends radially to the first axis X1 from the rest of the syringe barrel 10. The barrel flange 30 defines a thickness e30 measured along an axis parallel to the first axis X1 between a distal surface 35, perpendicular to the first axis X1 and directed towards the distal end 15, i.e. oriented in the injection direction D, and a proximal surface 40, perpendicular to the first axis X1 and opposed to the distal surface 35.

The injection device 1 comprises a cap 45. The cap 45 is movably mounted on the distal end 15. The cap 45 prevents fluid leakage and covers and protects the needle connector or the needle when the injection device 1 is not used.

The injection device 1 comprises a plunger rod 50 extending along a rod axis X50, which is superimposed with the first axis X1 when the injection device 1 is assembled. In other terms, the injection direction D extends along the rod axis X50. The plunger rod 50 comprises a rod portion 55 extending mainly along the rod axis X50 and a plunger flange 65. The plunger rod 50 is, for example, formed of a single part. The plunger rod 50 is, for example, an injection-molded part.

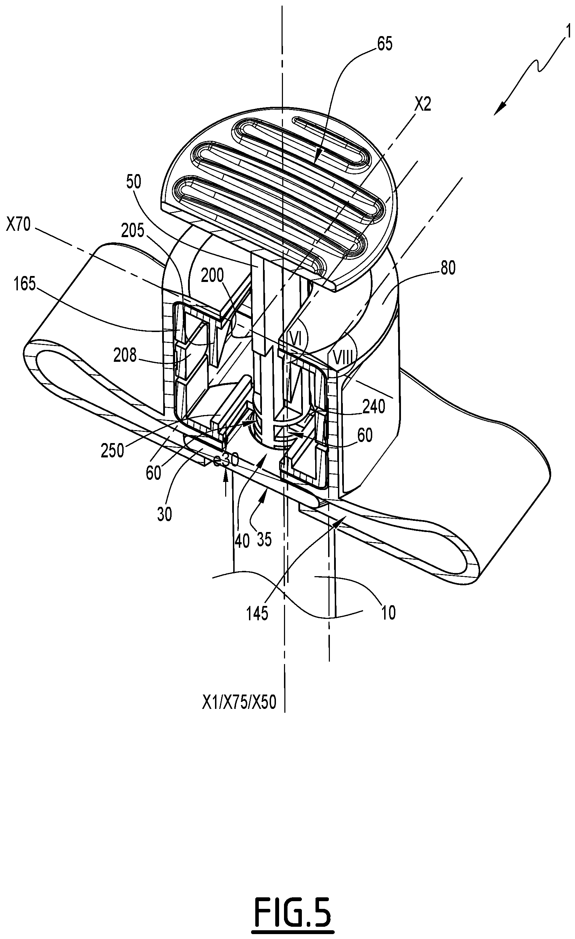

The rod portion 55 comprises, for example, at least one recess 60, in the illustrated embodiment two recesses 60, visible on Figure 5. The two recesses 60 are symmetrical with respect to the plan P comprising the rod axis X50. Each recess 60 runs through the rod portion 55, for example perpendicularly to the rod axis X50.

The plunger rod 50 comprises the plunger flange 65 positioned at a proximal end of the plunger rod 50. The plunger flange 65 is configured to be pressed by a finger of a user and extends, for example, perpendicularly to the rod axis X50.

Advantageously, the plunger rod 50 comprises at least one second pushing element 70, in the illustrated embodiment two second pushing elements 70. Advantageously, each second pushing element 70 is a pin 70. The pins 70 protrude from the rod portion 55 along a pin axis X70 different from the rod axis X50. For example, the pin axis X70 is perpendicular to the rod axis X50. Preferably, the two pins 70 extend in opposite directions from the rod portion 55.

When the injection device 1 is assembled, the plunger rod 50, in particular the rod portion 55, is received, at least partially, in the barrel volume 25 of the syringe barrel 10. More precisely, the plunger rod 50 is configured to translate, parallel to the rod axis X50 in the injection direction D, relative to the syringe barrel 10, from a first position to a second position. When the plunger rod 50 translates along the injection direction D, i.e. from the first position to the second position and from the second position to the third position, the fluid to inject and/or air bubbles are pushed from the barrel volume 25 to the needle and are expelled from the injection device 1 through the needle.

The injection device 1 comprises for example a stopper 72. The stopper 72 is received in the barrel volume 25, at a distal end of the rod portion 55, opposed to the proximal end. The stopper 72 is mobile in translation along the injection direction D relative to the syringe barrel 10. The stopper 72 has a maximum diameter, measured on a plan orthogonal to the rod axis X50, that is equal to a diameter of the barrel volume 25, measured on a plan orthogonal to the first axis X1, at least when the stopper 72 is received in the barrel volume 25. The stopper 72 is able to define a fluid sealing junction with the syringe barrel 10, in particular with the inner surface of the syringe barrel 10. In other words, when the stopper 72 is received in the barrel volume 25 and when a fluid is received in the barrel volume 25, between the stopper 72 and the distal end 15 of the syringe barrel 10, the fluid cannot pass between the stopper 72 and the syringe barrel 10, in particular the inner surface of the syringe barrel 10. The stopper 72 is able to cooperate with the plunger rod 50 to expel the fluid from the injection device 1. When the plunger rod 50 translates from the first position to the second position and from the second position to the third position, the rod portion 55 induces a movement of the stopper 72 in the injection direction D and the stopper pushes the fluid in the injection direction D. In one embodiment, the stopper 72 is fixed to the rod portion 55. Alternatively, the stopper 72 is detached from the rod portion 55.

The injection device 1 comprises a dosing system 75. Preferably, the dosing system 75 is symmetrical with respect to a plan P', which is coincident with the plan P, when the injection device 1 is assembled.

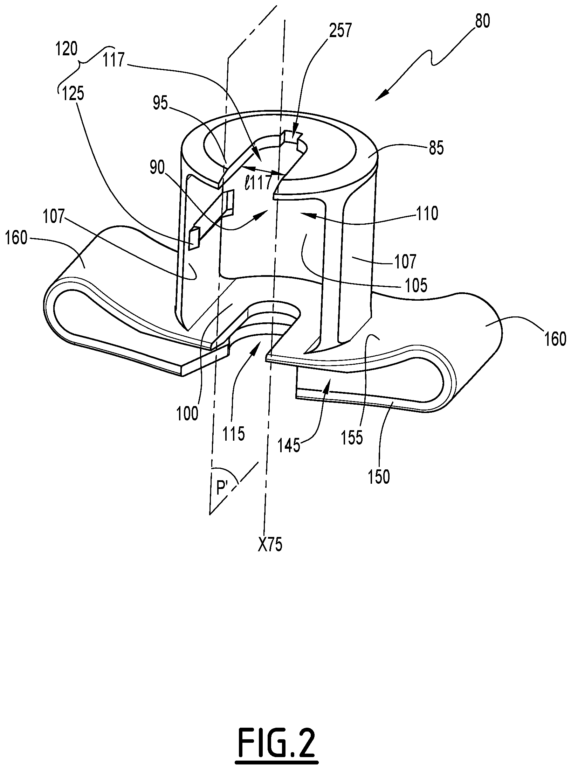

The dosing system 75 comprises a main part 80, represented alone on figure 2, configured to be assembled on the syringe barrel 10. The main part 80 comprises a body 85, for example tubular. The body 85 extends around a second axis X75, which is superimposed with the first axis X1 when the injection device 1 is assembled. The main part 80 is, for example, formed of a single part. The main part 80 is, for example, an injection-molded part.

The body 85 defines a receiving volume 90. In this embodiment, the receiving volume 90 is delimited by a first wall 95, advantageously perpendicular to the second axis X75, a second wall 100, advantageously perpendicular to the second axis X75 and offset along the injection direction D relative to the first wall 95, and a side wall 105 linking the first wall 95 to the second wall 100. The first wall 95 and respectively the second wall 100 have, for example, an overall disc shape. The receiving volume 90 is intended to receive partially the plunger rod 50. In other words, the receiving volume 90 is intended to receive a portion of the plunger rod 50.

The side wall 105 comprises, for example, two flat portions 107 parallel to the plan P' and preferably symmetrical to each other relative to the plan P'.

The side wall 105 defines an opening 110, leading into the receiving volume 90 and preferably symmetrical with respect to the plan P'.

The body 85 defines a first distal slot 115 and a first proximal slot 117. The first distal slot 115, respectively the first proximal slot 117, runs through the entire thickness of the second wall 100, respectively the first wall 95. The first distal slot 115 is aligned with first proximal slot 117 along a direction parallel to the second axis X75. The first distal slot 115, respectively, the first proximal slot 117 extends from an edge of the second wall 100, respectively, the first wall 95. The first distal slot 115, respectively the first proximal slot 117, is preferably symmetrical with respect to the plan P'. The distal slot 115, respectively the first proximal slot 117, extends from the second axis X75 to the opening 110 along a third axis X2, which is not parallel to the second axis X75, for example intersects the second axis X75. The third axis X2 is defined as the axis of sliding of a sliding part 165 of the dosing system relative to the main part 80. Advantageously, the third axis X2 is orthogonal to the second axis X75. In other words, the first distal slot 115, respectively the first proximal slot 117, extends the opening 110 in the respective first wall 95 and second wall 100.

Advantageously, the main part 80 comprises at least one first guiding element 120. The at least one first guiding element 120 comprises, for example, at least one first lateral guiding element 125 and/or the first proximal slot 117. In this example, the at least one first guiding element 120 comprises two first lateral guiding elements 125, preferably symmetrical to each other with respect to the plan P, and the first proximal slot 117. Advantageously, each first lateral guiding element 125 is a protrusion, extending on the side wall 105, for example, respectively on one of the flat portions 107. Each first lateral guiding element 125 extends from the side wall 105 into the receiving volume 90.

Advantageously, the main part 80 comprises a first contact surface 140 adapted to receive a finger of the user, more particularly to be pushed by a finger of a user. The first contact surface 140 extends on the side wall 105, preferably opposite to the opening 110.

Advantageously, the main part 80 comprises a housing 145 configured to receive the barrel flange 30 of the syringe barrel 10. The housing 145 comprises at least a first arm 150 and at least a second arm 155, advantageously two first arms 150 and two second arms 155. The two second arms 155 extends from the body 85, for example perpendicularly to the second axis X75. In the illustrated embodiment, the one part of the second arms 155 is formed by the second wall 100. Each first arm 150 is linked respectively, to a second arm 155 and extends, for example, parallel to the second arm 155. The housing 145 comprises at least one elastic element 160. In this example, the housing 145 comprises two elastic elements 160, each elastic element 160 linking, preferably symmetrically with respect to the plan P, a first arm 150 to a second arm 155. Each elastic element 160 is an elastic plastic layer, preferably made of a thermoplastic material, for example of a polyolefin such as polypropylene or polyethylene, a polycarbonate or a styrene polymer such as Acrylonitrile butadiene styrene. Alternatively, each elastic element 160 is made of a metallic material. Advantageously, the first arm 150, the second arm 155 and the elastic elements 160 are formed of a single part.

When the injection device 1 is assembled, the main part 80 is mounted on the syringe barrel 10, the barrel flange 30 being received in the housing 145. The first arms 150 are in contact with the distal surface 35. The second arms 155, pushed by the elastic elements 160 along the injection direction D, lean against the proximal surface 40 of the barrel flange 30. More precisely, when the main part 80 is mounted on the syringe barrel 10, the elastic elements 160 tends to bring closer the second arms 155 towards the first arms 150 along the second axis X75 so that the second arms 155 lean against the proximal surface 40. In this embodiment, the second arms 155 lean directly against the proximal surface 40 of the barrel flange 30. The elastic elements 160 exert a force on the first arms 150 and on the second arms 155 which tends to bring the first arms 150 and the second arms 155 closer together along the second axis X75. Hence, the elastic elements 160 ensures that a distance d160, measured along the first axis X1 between the first arms 150 and the second arms 155, is equal to the thickness e30 of the barrel flange 30 and the second arms 155 have a fixed position along a direction parallel to the first axis X1 compared to the distal surface 35 when the main part 80 is assembled on the syringe barrel 10, even when the injection device 1 is activated by a user. Thus, the elasticity of the elastic elements 160 allows the main part 80 to be assembled on barrel flanges 30 having different thicknesses whereas, when the barrel flange 30 is blocked in the housing 145, the syringe barrel 10 cannot move relative to the dosing system 75 along the first axis X1 so that the risk of error due to displacement of elements relative to one another is reduced. It is particularly advantageous with syringe barrel made of glass, for example according to ISO 11040-4 standard, which could have big tolerances in the barrel flange thickness.

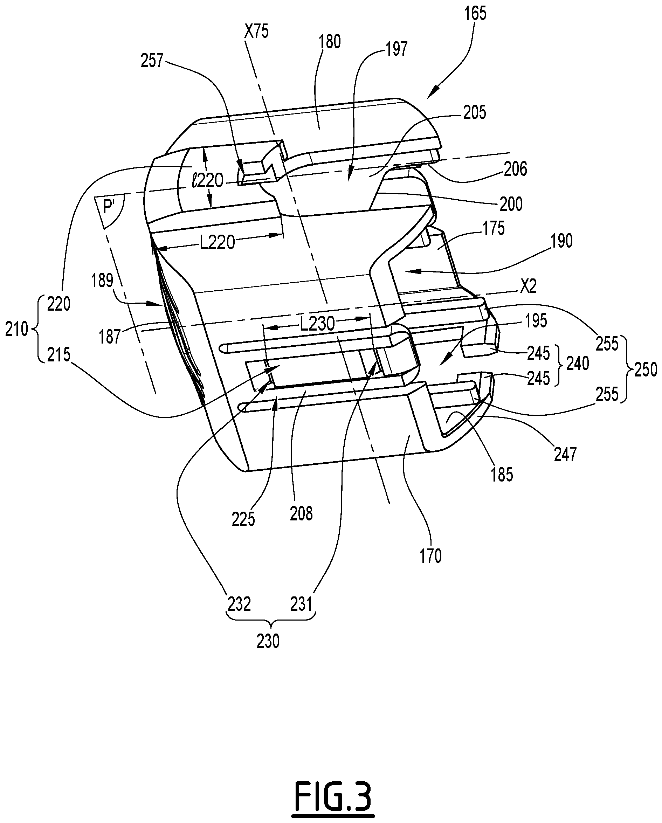

The dosing system 75 comprises the sliding part 165, represented on the figure 3. The sliding part 165 is configured to be mounted on the main part 80. In particular, the shape of the sliding part 165 is complementary to the receiving volume 90, so that the sliding part 165 is at least partially received in the receiving volume 90. The sliding part 165 is, for example, formed of a single part. The sliding part 165 is, for example, an injected-molded part.

In this example, the sliding part 75 comprises a first lateral wall 170 and a second lateral wall 175. The first lateral wall 170 and the second lateral wall 175, when the injection device 1 is assembled, are parallel to the plan P' and symmetrical to each other with respect to the plan P.

The sliding part 165 comprises an proximal wall 180, for example perpendicular to the first lateral wall 170 and to the second lateral wall 175, linking the first lateral wall 170 to the second lateral wall 175, and a distal wall 185, for example parallel to the proximal wall 180, offset along the injection direction D relative to the proximal wall 180 and linking the first lateral wall 170 to the second lateral wall 175.

The sliding part comprises a back wall 187 linking together the first lateral wall 170, the second lateral wall 175, the proximal wall 180 and the distal wall 185. Advantageously, the back wall 187 comprises a second contact surface 189 adapted to receive a finger of a user, more particularly to be pushed by a finger of a user.

The first lateral wall 170, the second lateral wall 175, the proximal wall 180, the distal wall 185 and the back wall 187 define an interior volume 190.

The proximal wall 180, respectively the distal wall 185, defines a second proximal slot 197 and, respectively a second distal slot 195. The second distal slot 195, respectively the second proximal slot 197, run through the entire thickness of the distal wall 185, respectively the proximal wall 180. The second distal slot 195 is aligned with second proximal slot 197 along a direction parallel to the second axis X75. The second distal slot 195, respectively, the second proximal slot 197 extends from an edge of the distal wall 185, respectively, the proximal wall 180. The second distal slot 195, respectively the second proximal slot 197, is preferably symmetrical with respect to the plan P'. The second distal slot 195, respectively the second proximal slot 197, extends from the second axis X75 to the opposite of the back wall 187 along the third axis X2. More precisely, the second distal slot 195, respectively the second proximal slot 197, is opened to the opposite of the back wall 187 along the third axis X2.

Advantageously, the sliding part 165 comprises at least one first pushing element 200, in the illustrated embodiment two first pushing elements 200, configured to cooperate with the plunger rod 50, notably with the at least one second pushing element 70, which is in the example a pin. Advantageously, each first pushing element is a ramp 200. The pin axis X70 is for example orthogonal to the third axis X2. In this embodiment, each ramp 200 is defined by an interior wall 205 of the sliding part 165. In other terms, the sliding part 165 comprises two interior walls 205 located in the interior volume 190 between the first lateral wall 170 and the second lateral wall 175 and each interior wall 205 defining one of the two ramps 200. The sliding part 165 comprises two interior walls 205 extending parallel to the first lateral wall 170 and the second lateral wall 175 and each interior wall 175 defines one of the ramps 200. The two interior walls 205 are, for example, symmetrical to each other with respect to the plan P'. The two interior walls 205 extends for example along a plane parallel to the third axis X2. The two interior walls 205 extends for example along a plane parallel to the second axis X75. When the injection device 1 is assembled, each ramp 200 is inclined by a first non-zero angle α with respect to the third axis X2. The first non-zero angle α is comprised between 10° and 75°, preferably between 30° and 60°, preferably equal to 45°. Each ramp 200 is inclined by a second non-zero angle β with respect to the second axis X75. The second non-zero angle β is comprised between 15° and 80°, preferably between 30° and 60°, preferably equal to 45°.The first non-zero angle α and the second non-zero angle β are measured on a plane parallel to the third axis X2 and parallel to the second axis X75, respectively between the ramp 200 and the projection of the third axis X2 and between the ramp 200 and the projection of the second axis X75.

Each interior wall 205 defines, for example, a proximal extension 206. The proximal extension 206 extends parallel to the third axis X2 and is continuous with an outer extremity of the ramp 200 opposed to the back wall 187. Each interior wall 205 defines a pin notch 207. The pin notch 207 extends parallel to the second axis X75 and is continuous with an inner extremity of the ramp 200, opposed to the outer extremity. The pin notch 207 is closer to the back wall 187 than the ramp 200. The ramp 200 is closer to the back wall 187 than the proximal extension 206.

Advantageously, the sliding part 165 comprises at least one elastic tongue 208, in the illustrated embodiment two elastic tongues 208. More precisely, the first lateral wall 170, respectively the second lateral wall 175, comprises one elastic tongue 208. The elastic tongues 208 are elastically deformable. The elastic tongues 208 extend, for example, parallel to the third axis X2. The elastic tongues 208 are disposed, preferably, with the back wall 187 at one of their extremities along the third axis X2 while their other extremity along the third axis X2 is free.

Advantageously, the sliding part 165 comprises at least one second guiding element 210. The at least one second guiding element 210 comprises for example at least one second lateral guiding elements 215 and/or one second proximal guiding element 220. In this example, the at least one second guiding element 210 comprises two second lateral guiding elements 215 and one second proximal guiding element 220. The two second lateral guiding elements 215 are, for example, symmetrical to each other with respect to the plan P. The second guiding elements 210 have complementary shapes to the first guiding elements 120 of the main part 80. More precisely, the second guiding elements 215 have respectively a complementary shape to the first guiding elements 125 and the second proximal guiding element 220 has a complementary shape to the first proximal slot 117.

Advantageously, each second lateral guiding element 215 is a groove, disposed, respectively, in one of the elastic tongues 208 and extending parallel to the third axis X2. Each groove is, for example, a blind groove. In the illustrated embodiment, each lateral guiding element 215 is disposed on an exterior surface 225, oriented to the opposite of the interior volume 190, of the corresponding elastic tongue 208. The second lateral guiding elements 215 are configured to receive the protrusions 125. Advantageously, each elastic tongue 208 also defines at least one notch 230, preferably in the second lateral guiding elements 215. In this example, the at least one notch 230 of each elastic tongue 208 comprises an activated notch 232, disposed in the second lateral guiding elements 215 near to the back wall 187, and an initial notch 231, disposed in the second lateral guiding elements 215 to the opposite of the back wall 187. The proximal guiding element 220 is a top protrusion, which protrudes from the proximal wall 180 towards the opposite of the interior volume 190. The top protrusion 220 has a shape complementary to the first proximal slot 117 of the first wall 95.

More precisely, the first proximal slot 117 defines a width ℓ117, measured perpendicularly to the first axis X1 and the third axis X2. The width ℓ117 is higher or equal to a width of the rod portion 55, measured perpendicularly to the rod axis X50 and to the third axis X2. Preferably, the width ℓ117 is equal to the width of the rod portion 55, more or less the mechanical clearances. In this way, the first proximal slot 117 guides the plunger rod 50 during its translation along the injection direction D. The top protrusion 220 defines a width ℓ220, measured perpendicularly to the first axis X1 and the third axis X2, equal to the width ℓ117 more or less the mechanical clearances. The top protrusion 220 defines a length L220, measured parallel to the third axis X2. The length L220 represents at least a stroke of the sliding part 165 from the initial position to the activated position.

Advantageously, the sliding part 165 comprises a first stop 240. The first stop 240 comprises at least one first stop wall 245, in the illustrated embodiment two first stop walls 245, preferably symmetrical to each other with respect to the plan P. Each first stop wall 245 is continuous with the distal wall 185 and preferentially extends in the same plan than the distal wall 185. Each first stop wall 245 extends from the distal wall 185 towards the second distal slot 195. The first stop walls 245 are positioned at a front extremity 247 of the distal wall 185 opposed to the back wall 187. The second distal slot 195 of the distal wall 185 is reduced at an extremity by the first stop walls 245.

The term "front", when referring to the sliding system 165, refers to a translation direction of the sliding system 165 along the third axis from the initial position to the activated position and refer to element oriented in the translation direction. The term "back", when referring to the sliding system 165, refers to a direction opposite to the translation direction. A "front surface" is oriented in the translation direction, a "back surface" is oriented in the opposite direction of the translation direction.

Advantageously, the injection device 5 and notably the dosing system 75 comprises a second stop 250. In this example, the sliding part 165 comprises the second stop 250. The second stop 250 comprises at least one second stop wall 255, in the illustrated embodiment two second stop walls 255, preferably symmetrical to each other with respect to the plan P. Each second stop wall 255 protrudes from the distal wall 185 into the interior volume 190, for example along a direction parallel to the second axis X75. Each second stop wall 255 extends from the back wall 187 to the front extremity 247, for example in a direction parallel to the third axis X2. Alternatively, the second stop 250 is formed by a portion of the distal wall 185 not protruding from the distal wall 185. In the example, each second stop is, respectively, a part of one of the interior walls 205. When the injection device 1 is assembled, the sliding part 165 is mounted in the receiving volume 90, the interior volume 190 leading into the receiving volume 90, and the first guiding elements 120 cooperate with the second guiding elements 210 to guide a translation, parallel to the third axis X2, of the sliding element 165 with respect to the main part 80. When the injection device 1 is assembled, the second contact surface 189 is opposed to the first contact surface 140, for example parallel to the first contact surface 140. The sliding part 165 is configured to slide along the third axis X2, from an initial position to an activated position. The sliding of the sliding part 165 is, for example, a pure translation. Advantageously, the third axis X2 is orthogonal to the first axis X1. When the sliding part 165 is in the initial position, the first lateral guiding elements 125, which are protrusions in this example, are received respectively in the initial notches 231. When the sliding part 165 is in the activated position, the protrusions 125 are received respectively in the activated notches 232. In other terms, the sliding part 165 is stabilized in its position relative to the main part 80 along the third axis X2, when the sliding part 165 is in the initial position or in the activated position. The sliding part 165 is, for example, stabilized only in the initial position and in the activated position.

When the injection device 1 is assembled, the elastic elements 160 are not functionally disposed between the sliding part 165 and the proximal surface 40. In this way, the elasticity provided by the elastic elements 160 does not impact the relative position of the sliding part 165 compared to the proximal surface 40, even when the injection device 1 is activated by a user. When the injection device 1 is assembled, the relative position of the sliding part 165 compared to the barrel flange 30 is fixed along a direction parallel to the first axis X1.

When the injection device 1 is assembled, the plunger rod 50 is preferably rotationally fixed around the rod axis X50 relative to the dosing system 75, preferably along at least the stroke of the plunger rod 50 between the first position to the third position. For example, the dosing system 75 comprises at least one anti-rotation element 257 able to cooperate with at least one complementary anti-rotation element 259 of the plunger rod 50 so as to prevent a rotation of the plunger rod 50 around the rod axis X50 relative to the dosing system 75. In the illustrated embodiment, the main part 80 and the sliding part 165 each comprise one anti-rotation element 257 and the plunger rod comprises two complementary anti-rotation elements 259, each anti-rotation element 257 is able to cooperate with respectively one complementary anti-rotation element 259. In the illustrated embodiment, each anti-rotation elements 257 is a slot, preferably extending along the third axis X2, defined respectively on the first wall 95 and on the proximal wall 180. In this example, the anti-rotation elements 257 are facing each other. Each complementary anti-rotation element 259 is a wall protruding from the rod portion 55 of the plunger rod 50.

Advantageously, the main part 80 and/or the sliding part 165 and/or the plunger rod 50 are made of a plastic material, preferably of a thermoplastic material, for example of a polyolefin such as polypropylene or polyethylene, of a polycarbonate or of a styrene polymer such as acrylonitrile butadiene styrene. Alternatively, the main part 80 and/or the sliding part 165 and/or the plunger rod 50 are made of a metallic material.

During the assembly of the injection device 1, the fluid to inject is filled in the barrel volume 25 of the syringe barrel 10. The barrel volume 25 receiving the fluid is then closed by the stopper 72. The plunger rod 50 is mounted on the syringe barrel 10 simultaneously to the stopper 72 or after the syringe stopper 72.

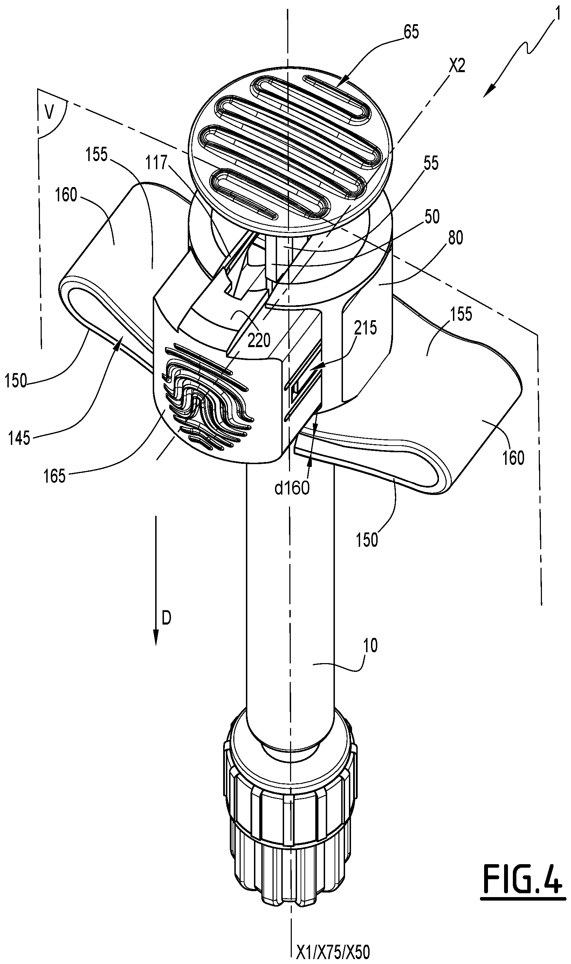

Then, the main part 80 is mounted on the syringe barrel 10, the plunger rod 50 being received in the first distal slot 115 and the first proximal slot 117 of the main part 80 so that the plunger rod is partially received in the receiving volume 90 defined by the body 85. The barrel flange 30 is received in the housing 145 as shown on the figures 1, 4 and 5. Then, the sliding part 165 is assembled with the main part 80 the plunger rod 50 being received in the second distal slot 195 and the second proximal slot 197, the first stop 240 being received in the recesses 60, and the sliding part 165 being in the initial position, as represented on the figures 1, 4 and 5. The plunger rod 50, the main part 80 and the sliding part 165 form an assembly 260. Thanks to the housing 145, the sliding part 165 have a fixed position along the first axis X1 compared to the distal surface 35 of the barrel flange 30 whatever the thickness e30 of the barrel flange.

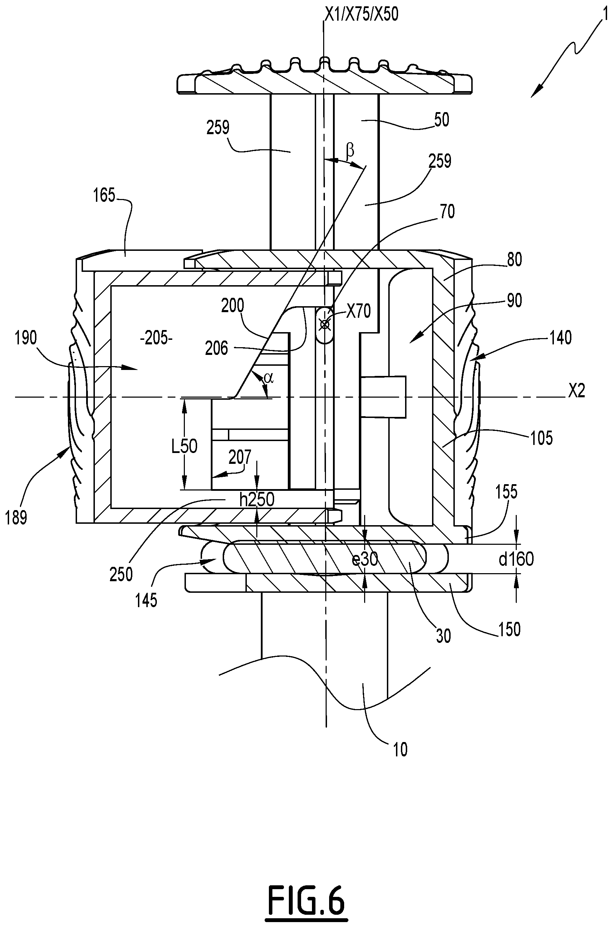

When the sliding part 165 is in the initial position, the first stop 240, in particular the first stop walls 245, is received in the recesses 60 of the plunger rod 50, thus blocking the plunger rod 50 in a first position, so that the initial position is stabilized, as represented on the figure 5. The translation of the plunger rod 50 with respect to the syringe barrel 10 is blocked so that there is no risk that the user moves unwittingly the plunger rod 50 by pressing the plunger flange 65 and spoils a part or the totality of the fluid. Moreover, even if the user wants to activate the injection device 1 by pressing the plunger flange 65 of the plunger rod 50, the plunger rod 50 will not translate. It reduces then the risk of misuse of the injection device 1. When the plunger rod 50 is in the first position, the pin 70 is in contact with the proximal extensions 206, as represented on the figure 6.

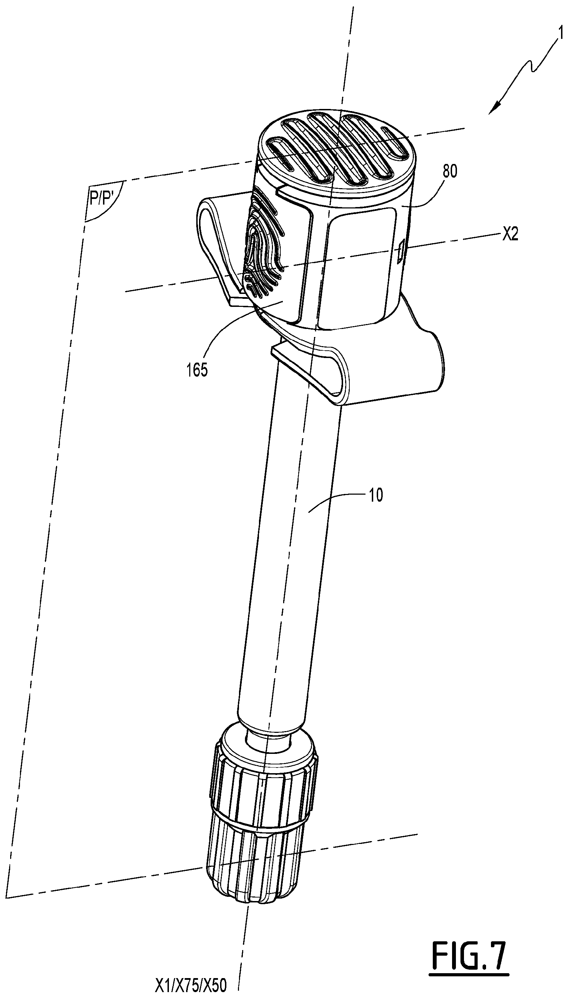

Then, to activate the injection device 1, the user pinches, between his fingers, the first contact surface 140 and the second contact surface 189 to slide the sliding part 165 from the initial position to the activated position, represented notably on the figure 7. When the user presses the first contact surface 140 and the second contact surface 189, the elastic tongues 208 are elastically deformed to allow the first lateral guiding elements 125, which are protrusions in this example, to exit the initial notches 231 to cooperate with the second lateral guiding elements 215, which are grooves in the example. The first lateral guiding elements 125 slide within the second lateral guiding elements 215 to guide the sliding of the sliding part 165 between the initial position and the activated position.

When the sliding part 165 slides from the initial position to the activated position, the plunger rod 50 cooperates with the sliding part 165 so that the plunger rod 205 translates relative to the body 85 and the syringe barrel 10, from the first position to a second position, in the injection direction D. The first position of the plunger rod 205 relative to the syringe barrel 10 corresponds to a first position relative to the main part 80 as the main part 80 is fixed relative to the syringe barrel 10 when the injection device 1 is assembled. More precisely, when the sliding part 165 slides from the initial position to the activated position, the pin 70 is in contact with the proximal extensions 206 and then with the ramps 200. When the sliding part 165 slides from the initial position to the activated position and the pin 70 is in abutment with the proximal extensions 206, the displacement of the sliding part 165 does not imply displacement of the plunger rod 50. More precisely, the sliding part 165 translates relative to the plunger rod 50 so that the first stop 240 runs through the recesses 60 until the first stop 240 is disengaged from the recesses 60. In other terms, in at least an intermediate position between the initial position and the activated position, the plunger rod 50 is received in the second distal slot 195 of the distal wall 185 and away from the two first stop walls 245 of the first stop 240, so that the plunger rod 50 is free to move in translation in the injection direction D relative to the dosing system 75. Then, the pin 70 is in abutment with the ramps 200. Thanks to the inclination of the ramps 200, the sliding of the sliding part 165 along the third axis X2 pushes the plunger rod 50 in translation in the injection direction D. The translation of the plunger rod 50 from the first position to the second position enables for example a purging step during which air bubbles are removed from the syringe barrel 10and the needle is filled with the fluid. Preferably, the injection device is oriented with the needle on top of the syringe barrel 10 during this purging step.

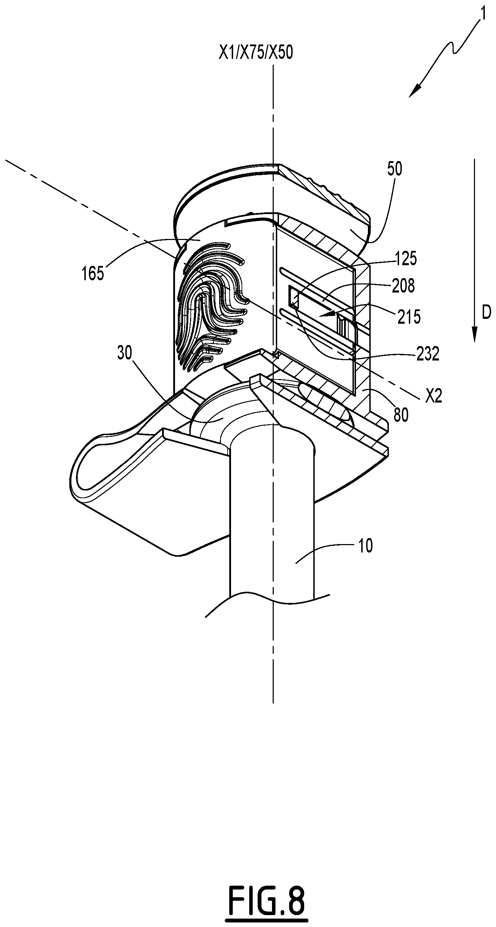

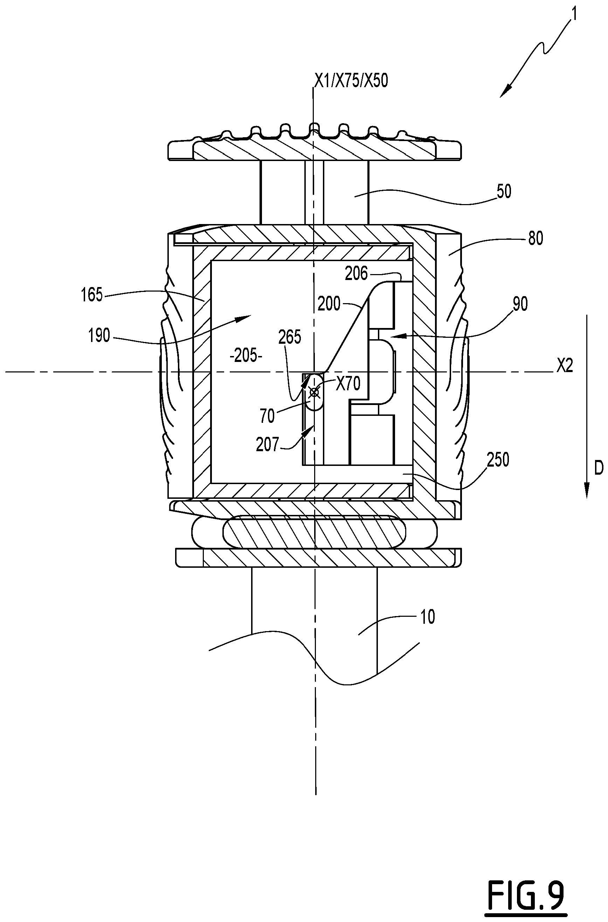

When the sliding part 165 reaches the activated position, as represented on figure 8, the elastic tongues 208 are elastically deformed to allow the first lateral guiding elements 125, which are protrusions in this example, to enter the activated notches 232 to block and stabilize the position of the sliding part 165 with respect to the main part 80. Thus, a stroke of the sliding part 165 from the initial position to the activated position is equal to a distance L230 measured parallel to the third axis X2 between the initial notch 231 and the activated notches 232. The length L220 of the top protrusion 220 is, for example, superior or equal to the distance L230 so that the sliding part 165 is efficiently guided by the proximal slot 117 and the top protrusion 220 over its entire stroke. When the sliding part 165 is in the activated position, the back wall 187 is preferably flush with the side wall 105. As shown on figure 9, when the sliding part 165 is in the activated position, the plunger rod 50 is in the second position and the pins 70 are received in the pin notches 207. The injection device 1 is ready for an injection step. Each pin notch 207 defines, for example, a horizontal surface 265 which extends parallel to the third axis X2 and is continuous with the respective ramp 200 so that once the pins 70 are received in the pin notches, the horizontal surfaces form an abutment to the pin 70 in a direction opposed to the injection direction D. Thus, the pin notches 70, in particular the horizontal surfaces 265, prevent a displacement of the plunger rod 50 in the direction opposed to the injection direction D from its second position towards its first position that could create air bubbles in the needle. Thus, the sliding of the sliding part 165 ensures a precise purging step, which can be performed prior to the injection step with no risk for air bubbles to form in the needle between the purging and the injection.

For the injection step, for example once the needle of injection device 1 is inserted in the target, the user presses the plunger flange 65 to pushes the plunger rod 50 in translation in the injection direction D to inject the fluid. During the injection step, the user maintains the syringe barrel 10 of the device stationary compared to the target, for example holding the main part 80 or the syringe barrel 10. The plunger rod 50 is blocked in its translation in a third position, beyond the second position in the injection direction D, by the second stop 250. In the third position, the pin 70 is in abutment with the second stop 250.

A volume of fluid injected to the target depends on an injection stroke L50 of the plunger rod 50. The injection stroke L50 is defined parallel to the rod axis X50 between the inner extremity of the ramp 200, in particular the horizontal surface 265, and the second stop 250. In other terms, the volume of fluid injected depends notably on a height h250 of the second stop 250 measured parallel to the first axis X1. The height h250 is calculated so that the translation of the plunger rod 50 in the injection direction D of the injection stroke L50 corresponds to a defined volume of fluid. Thus, thanks to the dosing system 75, in particular thanks to the second stop 250, the volume of fluid injected is accurate and does not depend on the user's appreciation. Therefore, the dosing system 75 corresponds to a certain volume of fluid to inject and the dosing system 75 can be chosen in a set, not represented, of dosing systems, each dosing system corresponding to a certain dose of injection. Advantageously, the dosing systems of the set can vary only by the height h250 of their respective second stop 250, i.e. by the travel of the pin 70.

The first position of the plunger rod 50 corresponds, for example, to a position of storage and/or start of purging step. The second position of the plunger rod 50 corresponds, for example, to a position of end of purging step and/or start of injection step. The third position of the plunger rod 50 corresponds, for example, to a position of end of injection step.

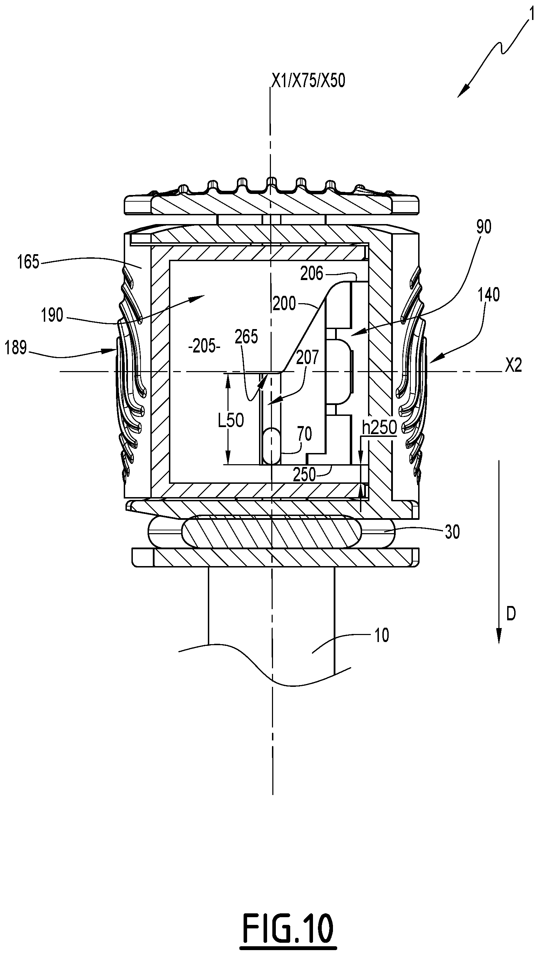

In the embodiment illustrated in figures 1 to 10, the main part 80 comprises two first lateral guiding elements 125, two first arms 150, two second arms 155, two elastic elements 160 the sliding part 165 comprises two ramps 200, two interior walls 205, two elastic tongues 208, two second lateral guiding elements 215, two first stop walls 245, two second stop walls 255 and the plunger rod 50 comprises two recesses 60. However, in alternative embodiments, the main part 80 comprises only one first lateral guiding element 125 and/or only one first arm 150 and/or only one second arm 155 and/or only one elastic element 160 and/or the sliding part 165 comprises only one ramp 200 and/or only one interior wall 205 and/or only one elastic tongue 208 and/or only one second lateral guiding element 215 and/or only one first stop wall 245 and/or only one second stop wall 255 and/or the plunger rod 50 comprises only one recesses 60. In these alternative embodiments, the above description applies the same way with only one element than with two elements.

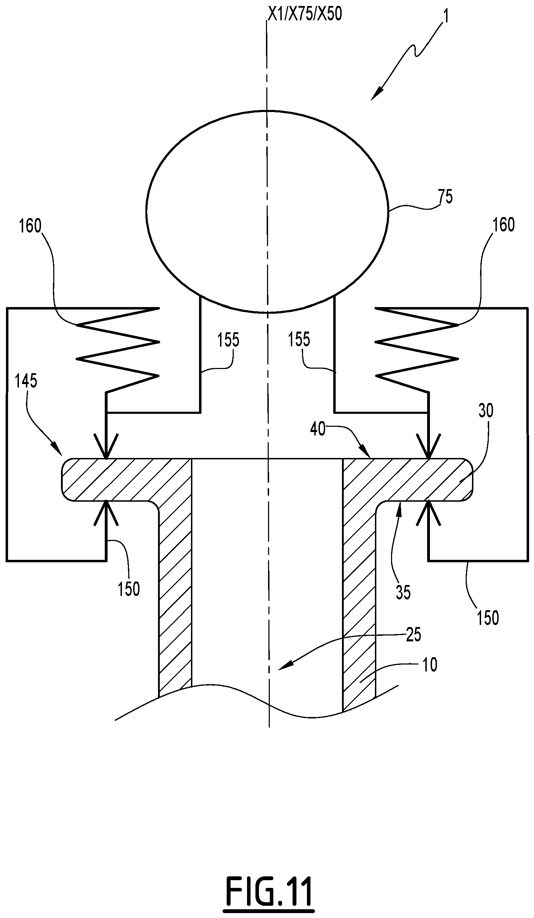

In the functional representation of figure 11, the elastic elements 160 are functionally represented by springs. The second arms 155 are linked to the first arms 150 thanks to the elastic element 160. More precisely, the first arms 150 and the second arms 155 have "U" shapes so that the barrel flange 30 is inside the "U" shapes when the dosing system 75 is mounted on the barrel flange 30.

The first arms 150 are represented extending above the second arms 155 according to a direction extending from the distal surface 35 to the proximal surface 40 along the first axis X1 so that the functioning of the elastic elements 160 is more understandable. Indeed, the elastic elements 160 are constrained, advantageously, between the first arms 150 and the second arms 155. More precisely, when the dosing system 75 is mounted on the barrel flange 30 of the syringe barrel 10, the first arms 150 are in contact with the distal surface 35 and the second arms 155 is in contact with the proximal surface 40. The elastic elements 160 exert a force on the first arms 150 and on the second arms 155 that tends to repel the second arms 155 towards the first arms 150 and the proximal face 40 to press the second arms 155 against the proximal surface 40. The elastic elements 160 enable a displacement of the second arms 155 with respect to the first arms 150 so that the housing 145 is adaptable to the size of the barrel flange 30.

In other terms, the elastic elements 160 allow a distance modification between the first and the second arms 150 and 155. The securing of the dosing system 75 is designed such as the dosing system 75 is directly linked to the proximal surface 40 of the barrel flange 30, without the intermediate of the elastic element 160. In this way, when the injection device 5 is assembled, the dosing system 75 position is always fixed compared to the barrel flange 30, even when the user applies pressure between the different elements of the injection device 5 for the actuation of the injection device 5.

The elastic elements 160 are preferably facing the proximal surface of the flange.

In a variant, each elastic elements 160 is a spring, preferably a helicoidal spring or a leaf spring.

In a variant, not represented, the first guiding element is a groove, the second guiding element is a protrusion configured to slide within the groove to guide the sliding of the sliding part between the initial position and the activated position and the first element is located on an elastic tongue. In other terms, the first guiding element and the second guiding element are inversed compared to the example of the figures.

In a variant, not represented, the sliding part is actuated by pulling parallel to the second axis.

In a variant, the main part comprises the second stop configured, when the sliding part is in the activated position, to prevent the plunger rod from being translated along the injection direction beyond the third position of the plunger rod. The second stop is for example formed by a portion of the second wall.

In a variant, the barrel flange 30, in particular the proximal surface 40 of the barrel flange 30, comprises the second stop configured, when the sliding part is in the activated position, to prevent the plunger rod from being translated along the injection direction beyond the third position of the plunger rod.

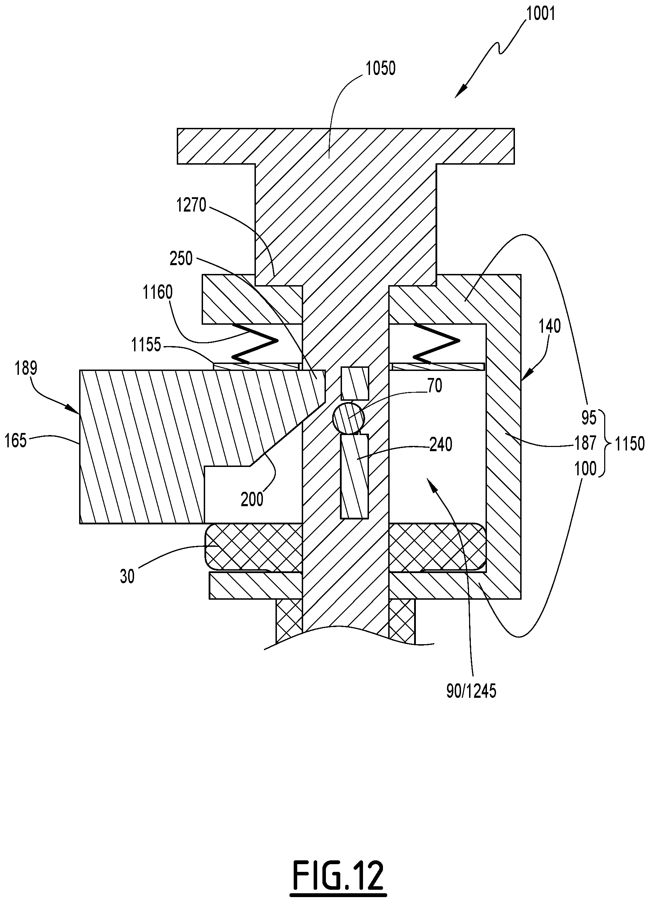

Figure 12 illustrates an injection device 1001 according to a second embodiment of the invention, the injection device 1001 being identical to the injection device 1 of the first embodiment except for the features described below. The reference signs for the injection device 1001 correspond to those of the injection device 1 when the referenced element is unchanged. The reference signs are increased by 1000 when they designate similar but modified elements in the injection device 1001. If a reference is mentioned in the remainder of the description without being shown on figure 12, or shown on figure 12 without being mentioned in the description, it relates to the element bearing the same reference in the first embodiment.

In the embodiment of figure 12, the housing 1145 and the receiving volume 90 are a single part volume. In other terms, the housing 1145 comprises a first arm 1150 including the first wall 95, the second wall 100 and the back wall 187.

The housing 1145 comprises a second arm 1155 and an elastic element 1160. The elastic element 1160 is for example a spring, preferably a helicoidal spring or a leaf spring or an elastic plastic layer. The elastic element 1160 is in the example, a helicoidal spring mounted around the plunger rod 1050. The second arm 1155 is linked to the first arm 1150 thanks to the elastic element 1160. The second arm 1155 is in contact with the proximal wall 180 of the sliding part 165. More precisely, the first arm 1150 has a "U" shape so that the barrel flange 30, the sliding part 165 and the second arm 1155 are inside the "U" shape when the dosing system 75 is mounted on the barrel flange 30.

The elastic element 1160 is constrained, advantageously, between the first arms 1150 and the second arms 1155. When the dosing system 75 is mounted on the barrel flange 30 of the syringe barrel 10, the first arm 1150, more precisely the second wall 100, is in contact with the distal surface 35 and the second arm 1155 leans against the sliding part 165 and the sliding part 165 is in contact with the proximal surface 40. In this embodiment, the second arm 1155 leans indirectly against the proximal surface 40 of the barrel flange 30, through the intermediate of the sliding part 165. More precisely, the elastic element 1160 exert a force on the first arm 1150 and on the second arm 1155 that tends to repel the second arm 1155 towards the second wall 100 by pressing the second arms 1155 against the sliding part 165 to press the sliding part 165 against the proximal surface 40. The elastic element 1160 enables a displacement of the second arm 1155 with respect to the first arm 1150 so that the housing 1145 is adaptable to the size of the flange 30.

In this embodiment, the second stop 250 is formed by the proximal wall 180 of the sliding part 165. The second stop 250 is in abutment with a step 1270 of the plunger rod 1050 when the plunger rod 1050 is in the third position. In an alternative embodiment, the second stop 250 is formed by the proximal surface 40 of the barrel flange 30.

It is to be understood that the plunger rod comprises a stopper configured to push the fluid in the injection direction D. Optionally, the plunger rod comprises a second pushing element configured to cooperate with a first pushing element of the sliding part so that, when the sliding part is sliding along the third axis from the initial position to the activated position, the plunger rod is pushed in the injection direction by the first pushing element from the first position to the second position. Optionally, the injection device comprises a first and a second stop configured to block the translation of the plunger rod depending on the position of the sliding part and to define the volume of fluid to inject. Optionally, the dosing system comprises an anti-rotation element able to cooperate with a complementary anti-rotation element of the plunger rod so as to prevent a rotation of the plunger rod around the rod axis relative to the dosing system. Optionally, once the injection device is assembled, the dosing system is dismountable from the syringe barrel and from the plunger rod.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the disclosure being set forth in the following claims.

## Claims

1. A dosing system (75) for an injection device (1;1001), the injection device comprising a syringe barrel (10) and a plunger rod (50; 1050) extending along a rod axis (X50), the syringe barrel (10) extending along a first axis (X1), the dosing system (75) comprising:
- a main part (80) configured to be assembled on the syringe barrel (10) of the injection device (1;1001), the main part (80) comprising a body (85) defining a receiving volume (90) which extends around a second axis (X75) and
- a sliding part (165) mounted on the main part (80) and configured to slide relative to the main part (80) along a third axis (X2), which intersects the second axis (X75), between an initial position and an activated position,
wherein, when the main part (80) is assembled on the syringe barrel (10) of the injection device (1; 1001), the receiving volume (90) is configured to receive at least partially the plunger rod (50; 1050) so that the second axis (X75), the rod axis (X50) and the first axis (X1) are superimposed, and the sliding part (165) is configured to cooperate with the plunger rod (50; 1050) when sliding along the third axis (X2) from the initial position to the activated position, so that the sliding part (165) pushes the plunger rod (50; 1050) in translation relative to the body (85), from a first position to a second position, in an injection direction (D), extending along the rod axis (X50).

2. Dosing system (75) according to claim 1, wherein the main part (80) comprises at least one first guiding element (120) and the sliding part (165) comprises at least one second guiding element (210), the at least one first guiding element (120) cooperating with the at least one second guiding element (210) to guide the sliding of the sliding part (165) along the third axis (X2) between the initial position and the activated position.

3. Dosing system (75) according to claim 2, wherein a first element of the at least one first guiding element (120) and the at least one second guiding element (210) comprises a groove (215) extending parallel to the third axis (X2) and a second element of the at least one first guiding element (120) and the at least one second guiding element (210) comprises a protrusion (125), the protrusion (125) being configured to slide within the groove (215) to guide the sliding of the sliding part (165) along the third axis (X2) between the initial position and the activated position.

4. Dosing system (75) according to claim 3, wherein the groove (215) comprises at least one notch (230) configured to receive the protrusion (125) so as to stabilize the sliding part (165) in the initial position and/or in the activated position.

5. Dosing system (75) according to claim 4 wherein the groove (215) is located on an elastic tongue (208), the tongue (208) being configured to elastically deform to allow the protrusion (125) to enter and exit the at least one notch (230).

6. Dosing system (75) according to any of the preceding claims, wherein the sliding part (165) comprises a first stop (240) configured to block the plunger rod (50; 1050) in the first position when the sliding part (165) is in the initial position.

7. Dosing system (75) according to any of the preceding claims, wherein the sliding part (165) comprises a second stop (250) configured, when the sliding part (165) is in the activated position, to prevent the plunger rod (50; 1050) from being translated along the injection direction (D) beyond a third position of the plunger rod (50; 1050), the third position being beyond the second position along the injection direction (D).

8. Dosing system (75) according to any of the preceding claims, wherein the main part (80) comprises a housing (145; 1145) configured to receive a barrel flange (30) of the syringe barrel (10), the housing comprising:
- a first arm (150; 1150) configured to be in contact with a distal surface (35) of the barrel flange (30),
- a second arm (155; 1155) secured to the body (85) of the main part (80) and configured to lean against a proximal surface (40) of the barrel flange (30), opposite to the distal surface (35), the second arm (155, 1155) being linked to the first arm (150; 1150) and mobile along a direction parallel to the second axis (X75) with respect to the first arm (150; 1150), and
- an elastic element (160; 1160), configured to exert a force onto the first arm (150; 1150) and the second arm (155; 1155) tending to bring the second arm (155; 1155) towards the first arm (150; 1150) along a direction parallel to the second axis (X75).

9. Dosing system (75) according to claim 8, wherein the first arm (150), the second arm (155) and the elastic element (160) are formed of a single part.

10. Dosing system (75) according to any one of the preceding claims, wherein, the second axis (X75) is orthogonal to the third axis (X2).

11. Dosing system (75) according to any of the preceding claims, wherein the main part (80) comprises a first contact surface (140) and the sliding part (165) comprises a second contact surface (189), opposed to the first contact surface (140) along the third axis (X2), the first and second contact surfaces (140, 189) being adapted to receive a finger of a user and being configured to allow the user to slide the sliding part (165).

12. Dosing system (75) according to any one of the previous claims, wherein the sliding part (165) comprises a ramp (200), inclined by a first non-zero angle (α) with respect to the third axis (X2) and inclined by a second non-zero angle (β) with respect to the second axis (X75), the ramp (200) being configured to cooperate with the plunger rod (50; 1050) so as to push the plunger rod (50; 1050) in translation relative to the syringe barrel (10) in the injection direction (D), from the first position to the second position, when the main part (80) is assembled on the syringe barrel (10) of the injection device (1;1001) and when the sliding part (165) is sliding along the third axis (X2) from the initial position to the activated position.

13. Assembly (260) of a dosing system (75) according to any one of the previous claims and a plunger rod (50; 1050), the plunger rod (50; 1050) extending along a rod axis (X50), the plunger rod (50; 1050) being configured to be received at least partially in the receiving volume (90) and to translate relative to the body (85) from a first position to a second position in the injection direction (D) extending along the rod axis (X50).

14. Assembly (260)according to claim 13, wherein :
- the sliding part (165) comprises a first pushing element (200), and
- the plunger rod (50; 1050) comprises;
∘ a rod portion (55), extending mainly along the rod axis (X50), and
∘ a second pushing element (70) configured to cooperate with the first pushing element (200) so that, when the sliding part (165) is sliding along the third axis (X2) from the initial position to the activated position, the plunger rod (50; 1050) is pushed in the injection direction (D) by the first pushing element (200) from the first position to the second position.

15. Assembly (260) according to any of the claims 13 or 14 wherein:
- the first pushing element (200) is a ramp (200), inclined by a first non-zero angle (α) with respect to the third axis (X2) and inclined by a second non-zero angle (β) with respect to the second axis (X75), and
- the second pushing element is a pin (70), protruding from the rod portion (55) along a pin axis (X70) distinct from the rod axis (X50).

16. The assembly (260) according to any one of the claims 14 or 15, wherein:
- the sliding part (165) comprises a second stop (250), wherein, when the sliding part (165) is in the activated position, the second stop (250) is configured to prevent the plunger rod (50; 1050) from being translated along the injection direction (D) beyond a third position of the plunger rod (50; 1050), the third position being beyond the second position along the injection direction (D), and
- the second pushing element (70) of the plunger rod (50; 1050) is in abutment with the second stop (250) of the dosing system (75) along the injection direction (D) when the plunger rod (50; 1050) is in the third position and the sliding part (165) is in the activated position.

17. Injection device (1;1001) comprising:
- a syringe barrel(10)extending along a first axis (X1) and
- an assembly (260) according to any one of the claims 13 to 16,
wherein the main part (80) of the dosing system (75) is assembled on the syringe barrel (10) and the plunger rod (50; 1050) is mounted on the syringe barrel (10) and the plunger rod (50; 1050) is at least partially received in the receiving volume (90).

## Amended claims

### Amended claims in accordance with Rule 137(2) EPC.

1. A dosing system (75) for an injection device (1;1001), the injection device comprising a syringe barrel (10) and a plunger rod (50; 1050) extending along a rod axis (X50), the syringe barrel (10) extending along a first axis (X1), the dosing system (75) comprising:
- a main part (80) configured to be assembled on the syringe barrel (10) of the injection device (1;1001), the main part (80) comprising a body (85) defining a receiving volume (90) which extends around a second axis (X75) and
- a sliding part (165) mounted on the main part (80) and configured to slide relative to the main part (80) along a third axis (X2), which intersects the second axis (X75), between an initial position and an activated position,
wherein, when the main part (80) is assembled on the syringe barrel (10) of the injection device (1; 1001), the receiving volume (90) is configured to receive at least partially the plunger rod (50; 1050) so that the second axis (X75), the rod axis (X50) and the first axis (X1) are superimposed,
the dosing system being **characterized in that** the sliding part (165) is configured to cooperate with the plunger rod (50; 1050) when sliding along the third axis (X2) from the initial position to the activated position, so that the sliding part (165) pushes the plunger rod (50; 1050) in translation relative to the body (85), from a first position to a second position, in an injection direction (D), extending along the rod axis (X50).

2. Dosing system (75) according to claim 1, wherein the main part (80) comprises at least one first guiding element (120) and the sliding part (165) comprises at least one second guiding element (210), the at least one first guiding element (120) cooperating with the at least one second guiding element (210) to guide the sliding of the sliding part (165) along the third axis (X2) between the initial position and the activated position.

3. Dosing system (75) according to claim 2, wherein a first element of the at least one first guiding element (120) and the at least one second guiding element (210) comprises a groove (215) extending parallel to the third axis (X2) and a second element of the at least one first guiding element (120) and the at least one second guiding element (210) comprises a protrusion (125), the protrusion (125) being configured to slide within the groove (215) to guide the sliding of the sliding part (165) along the third axis (X2) between the initial position and the activated position.

4. Dosing system (75) according to claim 3, wherein the groove (215) comprises at least one notch (230) configured to receive the protrusion (125) so as to stabilize the sliding part (165) in the initial position and/or in the activated position.

5. Dosing system (75) according to claim 4 wherein the groove (215) is located on an elastic tongue (208), the tongue (208) being configured to elastically deform to allow the protrusion (125) to enter and exit the at least one notch (230).

6. Dosing system (75) according to any of the preceding claims, wherein the sliding part (165) comprises a first stop (240) configured to block the plunger rod (50; 1050) in the first position when the sliding part (165) is in the initial position.

7. Dosing system (75) according to any of the preceding claims, wherein the sliding part (165) comprises a second stop (250) configured, when the sliding part (165) is in the activated position, to prevent the plunger rod (50; 1050) from being translated along the injection direction (D) beyond a third position of the plunger rod (50; 1050), the third position being beyond the second position along the injection direction (D).

8. Dosing system (75) according to any of the preceding claims, wherein the main part (80) comprises a housing (145; 1145) configured to receive a barrel flange (30) of the syringe barrel (10), the housing comprising:
- a first arm (150; 1150) configured to be in contact with a distal surface (35) of the barrel flange (30),
- a second arm (155; 1155) secured to the body (85) of the main part (80) and configured to lean against a proximal surface (40) of the barrel flange (30), opposite to the distal surface (35), the second arm (155, 1155) being linked to the first arm (150; 1150) and mobile along a direction parallel to the second axis (X75) with respect to the first arm (150; 1150), and
- an elastic element (160; 1160), configured to exert a force onto the first arm (150; 1150) and the second arm (155; 1155) tending to bring the second arm (155; 1155) towards the first arm (150; 1150) along a direction parallel to the second axis (X75).

9. Dosing system (75) according to claim 8, wherein the first arm (150), the second arm (155) and the elastic element (160) are formed of a single part.

10. Dosing system (75) according to any one of the preceding claims, wherein, the second axis (X75) is orthogonal to the third axis (X2).

11. Dosing system (75) according to any of the preceding claims, wherein the main part (80) comprises a first contact surface (140) and the sliding part (165) comprises a second contact surface (189), opposed to the first contact surface (140) along the third axis (X2), the first and second contact surfaces (140, 189) being adapted to receive a finger of a user and being configured to allow the user to slide the sliding part (165).

12. Dosing system (75) according to any one of the previous claims, wherein the sliding part (165) comprises a ramp (200), inclined by a first non-zero angle (α) with respect to the third axis (X2) and inclined by a second non-zero angle (β) with respect to the second axis (X75), the ramp (200) being configured to cooperate with the plunger rod (50; 1050) so as to push the plunger rod (50; 1050) in translation relative to the syringe barrel (10) in the injection direction (D), from the first position to the second position, when the main part (80) is assembled on the syringe barrel (10) of the injection device (1;1001) and when the sliding part (165) is sliding along the third axis (X2) from the initial position to the activated position.

13. Assembly (260) of a dosing system (75) according to any one of the previous claims and a plunger rod (50; 1050), the plunger rod (50; 1050) extending along a rod axis (X50), the plunger rod (50; 1050) being configured to be received at least partially in the receiving volume (90) and to translate relative to the body (85) from a first position to a second position in the injection direction (D) extending along the rod axis (X50).

14. Assembly (260)according to claim 13, wherein :
- the sliding part (165) comprises a first pushing element (200), and
- the plunger rod (50; 1050) comprises;
∘ a rod portion (55), extending mainly along the rod axis (X50), and
∘ a second pushing element (70) configured to cooperate with the first pushing element (200) so that, when the sliding part (165) is sliding along the third axis (X2) from the initial position to the activated position, the plunger rod (50; 1050) is pushed in the injection direction (D) by the first pushing element (200) from the first position to the second position.

15. Assembly (260) according to claim 14 wherein:
- the first pushing element (200) is a ramp (200), inclined by a first non-zero angle (α) with respect to the third axis (X2) and inclined by a second non-zero angle (β) with respect to the second axis (X75), and
- the second pushing element is a pin (70), protruding from the rod portion (55) along a pin axis (X70) distinct from the rod axis (X50).

16. The assembly (260) according to any one of the claims 14 or 15, wherein:
- the sliding part (165) comprises a second stop (250), wherein, when the sliding part (165) is in the activated position, the second stop (250) is configured to prevent the plunger rod (50; 1050) from being translated along the injection direction (D) beyond a third position of the plunger rod (50; 1050), the third position being beyond the second position along the injection direction (D), and
- the second pushing element (70) of the plunger rod (50; 1050) is in abutment with the second stop (250) of the dosing system (75) along the injection direction (D) when the plunger rod (50; 1050) is in the third position and the sliding part (165) is in the activated position.

17. Injection device (1;1001) comprising:
- a syringe barrel(10)extending along a first axis (X1) and
- an assembly (260) according to any one of the claims 13 to 16,
wherein the main part (80) of the dosing system (75) is assembled on the syringe barrel (10) and the plunger rod (50; 1050) is mounted on the syringe barrel (10) and the plunger rod (50; 1050) is at least partially received in the receiving volume (90).
